# EUROPÄISCHE PATENTANMELDUNG

(11) **EP 4 670 681 A1**
(43) Veröffentlichungstag der Anmeldung: **31.12.2025**
(21) Anmeldenummer: 24184191.5
(22) Anmeldetag: 25.06.2024
(51) Int. Cl.: A61F 2/30, A61F 2/46

(54) **GELENKIMPLANTAT UND ZUGEHÖRIGE VORRICHTUNGEN, SYSTEM UND VERFAHREN**

(71) Anmelder: Heraeus Medical GmbH, 61273 Wehrheim (DE)
(72) Erfinder: VOGT, Sebastian, 61273 Wehrheim (DE); KLUGE, Thomas, 61273 Wehrheim (DE)
(74) Vertreter: Heraeus IP

(57) **Zusammenfassung**

Die Erfindung betrifft ein Gelenkimplantat zum Freihalten einer Öffnung in einem Gelenkknochen, zwei Vorrichtungen zum Setzen eines Gelenkimplantats, ein System und ein Verfahren zum Setzen eines Gelenkimplantats. Ein Gelenkimplantat (10) zum Freihalten einer Öffnung (2) in einem Gelenkknochen (5) umfasst einen länglichen Körper (12), der sich entlang einer Längsachse (15) erstreckt, wobei der Körper (12) entlang der Längsachse (15) einen durchgehenden Kanal (16) definiert. An einer Außenseite (14) des Körpers (12) ist mindestens ein Verankerungselement (20) zum Verankern des Gelenkimplantats (10) in dem Gelenkknochen (5) angeordnet.

## Beschreibung

Die Erfindung betrifft ein Gelenkimplantat zum Freihalten einer Öffnung in einem Gelenkknochen, zwei Vorrichtungen zum Setzen eines Gelenkimplantats, ein System und ein Verfahren zum Setzen eines Gelenkimplantats.

In Gelenken treten Knorpelschäden auf. Diese sind häufig mit Schmerzen und einer Beeinträchtigung der Gelenkfunktion verbunden. Knorpel sind nicht durchblutet. Der Stofftransport zur Nährstoffversorgung sowie zur Abführung von Stoffwechselendprodukten erfolgt durch die Gelenkflüssigkeit und die Knorpelhaut. Die natürliche Regeneration von Knorpelschäden ist deshalb begrenzt.

Eine bekannte Therapie bei Knorpelschäden ist die Mikrofrakturierung nach K. H. Pridie. Hierbei wird die die unter dem Knorpel befindliche subchondrale Knochenplatte mittels einer oder mehrerer Bohrungen geöffnet, so dass Blut und Knochenmark aus dem darunter liegenden Gewebe des Knochens durch die Öffnung zum Knorpel gelangen können. Auf diese Weise kann der Knorpel verbessert versorgt werden und es können nicht benötigte Produkte abgeführt werden. Typischerweise wird durch die zyklischen Be- und Entlastungsphasen und damit einhergehende Kompression und Dekompression des Gelenks ein zyklischer Stoffstrom in abwechselnden Richtungen durch die hergestellte Öffnung erzeugt. Die Selbstheilung des Knorpels wird so ermöglicht bzw. verbessert.

Eine Weiterentwicklung dieses Verfahrens stellt die autologe matrixinduzierte Chondrogenese nach P. Behrens dar, die auch als matrixgekoppelte Mikrofrakturierung bezeichnet wird. Hierbei wird der zu behandelnde Knorpeldefekt zusätzlich an der Oberseite mit einer zweischichtigen Kollagenmembran überdeckt.

Beiden Verfahren ist gemein, dass die Wirkung nach einiger Zeit nachlässt. Es wird davon ausgegangen, dass dies durch ein natürliches Verschließen bzw. Zuwachsen der hergestellten Öffnung bedingt ist. Dieser Effekt kann zwar durch z. B. eine Ultraschallbehandlung verzögert oder temporär rückgängig gemacht werden, allerdings sind dann regelmäßige Behandlungen notwendig. Zudem wird die Wirkung solcher Behandlungen mit der Zeit schwächer.

Die Druckschrift EP 3 801 394 B1 offenbart ein Gelenkimplantat zur Gewebeneubildung am Gelenk, das aus einem stiftförmigen Körper mit einer Gewindestruktur und einer 3D-gedruckten künstlichen Trabekularstruktur besteht. Auf diese Weise soll ein rasches Überwachsen angeregt werden.

Die Aufgabe der Erfindung besteht darin, ein Gelenkimplantat, mit dem die Selbstheilung eines geschädigten Knorpels möglichst langfristig sichergestellt werden kann, sowie zugehörige Vorrichtungen, ein System und ein Verfahren zur Verfügung zu stellen.

Die Aufgabe wird gelöst durch das Gelenkimplantat gemäß Anspruch 1 und die Vorrichtungen, das System sowie das Verfahren gemäß den nebengeordneten Ansprüchen. Vorteilhafte Ausgestaltungen sind in den Unteransprüchen angegeben.

Zur Lösung der Aufgabe dient ein Gelenkimplantat zum Freihalten einer Öffnung in einem Gelenkknochen, das einen insbesondere länglichen Körper umfasst, der sich entlang einer Längsachse erstreckt. Der Körper definiert entlang der Längsachse einen durchgehenden Kanal. An einer Außenseite des Körpers ist mindestens ein Verankerungselement zum Verankern des Gelenkimplantats in einem Gelenkknochen angeordnet.

Die Erfindung beruht auf der Erkenntnis, dass eine möglichst dauerhafte Fluidleitung zwischen dem zu regenerierenden Knorpel und dem subchondralen Raum, in dem sich Blut und Knochenmark bzw. Knochengewebe befinden, die Funktion und Regeneration des Knorpels dauerhaft sicherstellen kann. Das erfindungsgemäße Gelenkimplantat stellt durch den durchgehenden Kanal eine langlebige Fluidverbindung bereit, die durch die subchondrale Knochenplatte hindurch verläuft. Das Gelenkimplantat kann in bereits bekannte Therapien wie die Mikrofrakturierung und die autologe matrixinduzierte Chondrogenese integriert. Das Gelenkimplantat kann in eine hergestellte Öffnung bzw. Bohrung eingesetzt werden und verbleibt aufgrund des Verankerungselements am Knochen verankert.

Der Gelenkknochen ist ein Knochen im Bereich des Gelenks. Insbesondere ist die subchondrale Knochenplatte gemeint. Insbesondere ist das Gelenkimplantat so bemessen und/oder wird das Gelenkimplantat so eingesetzt, dass sich ein distales Ende des Gelenkimplantats auf Höhe der Unterkante der subchondralen Knochenplatte befindet oder über diese hervorsteht und/oder ein proximales Ende des Gelenkimplantats auf oder unterhalb der Unterkante des Knorpels bzw. chondralen Bereichs angeordnet ist. Es ist vorteilhaft, wenn das distale Ende des Implantats etwas über den subchondralen Knochen herausragt. Das proximale Ende sollte hingegen nicht in den Knorpel hineinragen, um die Gelenkfunktion nicht zu beeinträchtigen und um selbst nicht beschädigt zu werden.

Der Körper ist insbesondere länglich. Das bedeutet, dass die axiale Erstreckung des Körpers größer ist als ein senkrecht dazu gemessener Durchmesser des Körpers, beispielsweise um einen Faktor von wenigstens 2. In einer Ausführungsform beträgt das Verhältnis von axialer Länge des Gelenkimplantats bzw. des Körpers zum äußeren Durchmesser des Gelenkimplantats bzw. des Körpers wenigstens 3:1 und/oder höchstens 7:1.

Der Körper kann eine kreiszylindrische Grundform aufweisen. Der Kanal kann zumindest in Abschnitten einen Kreisquerschnitt aufweisen. Bevorzugt ist der Körper und/oder der Kanal geradlinig geformt. Dies erleichtert das Einbringen bzw. die Durchströmung. Insbesondere enthält der Kanal keine Biegungen oder Knicke.

Der Kanal ist durchgehend. Das heißt, er verläuft in axialer Richtung vollständig durch das Gelenkimplantat hindurch. Der Kanal ist offen. Auf diese Weise kann eine durchgehende Verbindung zwischen zwei Bereichen hergestellt werden, die sich axial beidseitig des Gelenkimplantats befinden. Axial bezieht sich auf die Längsachse.

Das Verankerungselement befindet sich bevorzugt an der radialen Außenseite. Das Verankerungselement steht insbesondere radial über benachbarte Regionen der Außenseite hervor. Das Verankerungselement dient dazu, ein Verschieben des Gelenkimplantats im Knochen, insbesondere entlang der Längsachse, zu verhindern. Das Verankerungselement kann beispielsweise eine lokale Verbreiterung sein, etwa in Form eines konvexen Formelements. Auch bei Bildung von Druck und/oder von Flüssigkeitsströmung durch den Kanal bei zyklischer Belastung wird das Gelenkimplantat so in seiner Position gehalten. Es können mehrere Verankerungselemente vorhanden sein, um verbessert und/oder an mehreren Stellen eine Verankerung herzustellen.

In einer Ausführungsform umfasst der Körper ein biokompatibles Metall oder eine biokompatible Metalllegierung oder ist daraus hergestellt, beispielsweise umfassend Tantal, Titan, Cobalt-Chrom-Stahl, 316L-Stahl und/oder amorphes Metall (metallisches Glas). Bevorzugt sind die Legierung TiAl6V4, der Edelstahl 1.4404, Edelstahl 1.4403, Palladium, Palladium-Legierungen, Platin und/oder Platinlegierungen. Wenn der Körper ferromagnetisches Metall und/oder magnetisches, amorphes Metall umfasst oder daraus hergestellt ist, kann der Kanal durch Magnetostriktion infolge des Anlegens eines oder mehrerer magnetischer Wechselfelder gereinigt werden. Ein diesbezügliches Verfahren zur Reinigung des Gelenkimplantats umfasst demnach das Aufbringen eines oder mehrerer magnetischer Wechselfelder auf den Körper.

In einer Ausführungsform umfasst der Körper einen biokompatiblen Kunststoff oder ist daraus hergestellt, beispielsweise Polyetherketon, Polyethersulfon, Polyamid 12, Polyetherimid und/oder Polyamidimid. Beispielsweise kann ein hydrophober Bereich des Körpers aus einem solchen Material hergestellt sein.

In einer Ausgestaltung weist der durchgehende Kanal an einer engsten Stelle eine freie Querschnittsfläche von kleiner oder gleich 0,2 mm² auf.

Die freie Querschnittsfläche ist die Fläche, die ein Fluid zur Verfügung hat, um durch den Kanal durch das Gelenkimplantat hindurchzuströmen. Im einfachsten Fall eines Kanals mit Kreisquerschnitt entspricht die freie Querschnittsfläche einer Kreisfläche, die durch den inneren Durchmesser des Kanals definiert ist. Die freie Querschnittsfläche kann allerdings von festen oder beweglichen Teilen des Gelenkimplantats eingeschränkt sein, beispielsweise von Verengungen im Kanal und/oder von einem oder mehreren Scherkörpern wie unten beschrieben. Beispielsweise im Falle eines kreiszylinderförmigen Kanals mit einem darin enthaltenen Scherkörper ist die engste Stelle die Stelle, an der der Scherkörper den größten Querschnitt aufweist.

Beträgt die freie Querschnittsfläche im Falle eines Kanals mit Kreisquerschnitt ohne Verengungen oder Scherelemente 0,2 mm², entspricht dies einem Durchmesser des Kanals von etwa 500 µm. Die freie Querschnittsfläche kann kleiner oder gleich 0,07 mm² sein. Dies entspricht im oben beschriebenen Fall einem Durchmesser von ca. 300 µm. Die freie Querschnittsfläche beträgt typischerweise mindestens 0,001 mm² und bevorzugt mindestens 0,01 mm².

Der Durchmesser des Kanals beträgt insbesondere wenigstens 20 µm und/oder höchstens 2000 µm, bevorzugt höchstens 400 µm. Dadurch wird ein Einwachsen von Knochengewebe in den Kanal weitgehend unwahrscheinlich. Der Außendurchmesser des Körpers beträgt insbesondere wenigstens 500 µm und/oder höchstens 2500 µm.

Es hat sich gezeigt, dass bei einem Durchmesser ab etwa 300 µm Knochengewebe einwachsen kann. Dies soll verhindert werden, um die Fluidleitung möglichst dauerhaft zu ermöglichen. Es ist also zumindest eine Stelle vorhanden, in der Knochenmark nicht einwachsen kann. Auf der anderen Seite ermöglicht ein größerer Querschnitt einen größeren Stofftransport. Die 300 µm sind dabei keine starre Grenze, sondern das Einwachsen hängt von Rahmenbedingungen ab, wie beispielsweise von der Oberflächenbeschaffenheit im Bereich der Öffnung und den Strömungs- und Belastungsbedingungen. Bereits bei einem Durchmesser von etwa 500 µm kann unter günstigen Bedingungen ein Einwachsen langfristig verhindert werden.

Der Kanal hat bevorzugt eine solche Querschnittsfläche, dass zelluläre Bestandteile des Blutes, wie Leukozyten mit z. B. einem Durchmesser im Bereich von 12 µm bis 30 µm und scheibenförmigen Erythrozyten mit z. B. einem Durchmesser von 6,2 µm bis 8,2 µm den Kanal durchqueren können. Es wird davon ausgegangen, dass die flüssigen und zellulären Bestandteile des Blutes zur Versorgung und Regeneration des Knorpelgewebes beitragen können.

In einer Ausführungsform beträgt eine axiale Länge des Gelenkimplantats wenigstens 2 mm, bevorzugt wenigstens 3 mm. In einer Ausführungsform beträgt eine axiale Länge des Gelenkimplantats höchstens 12 mm, insbesondere höchstens 10 mm, bevorzugt höchstens 8 mm, besonders bevorzugt höchstens 7 mm. Die Länge ist typischerweise dergestalt, dass der Knochen im Bereich des Gelenks, insbesondere die subchondrale Knochenplatte vollständig durchdrungen werden kann. Die subchondrale Knochenplatte ist im Bereich der humanen Gelenke sehr dünn und hat eine Dicke von etwa 2 mm bis 5 mm. Es kann ein gewisses Übermaß vorgesehen sein, um die Handhabung und das Setzen zu erleichtern. Insbesondere ist die Länge des Gelenkimplantats nicht mehr als 2 mm größer als die Dicke der subchondralen Platte. Steht nur unten über.

In einer Ausführungsform ist die den Kanal definierende Innenwand der Körpers und/oder eine distale stirnseitige Fläche des Körpers aus Kunststoff hergestellt. In einer Ausführungsform ist die den Kanal definierende Innenwand der Körpers und/oder eine distale stirnseitige Fläche des Körpers hydrophob, hydrophob nanostrukturiert und/oder mit einer hydrophoben Beschichtung versehen.

Auch die hydrophobe Ausführung verhindert ein An- bzw. Einwachsen von Knochengewebe im Kanal. So kann eine möglichst dauerhafte Durchlässigkeit erreicht werden. Stammzellen oder Osteoblasten, Proteine und auch Zellen können sich an hydrophoben Oberflächen nicht oder nur schlecht anlagern. Insbesondere ist eine Hydrophobie oder Superhydrophobie gemeint, also ein Randwinkel von etwa 90° oder größer. Eine hydrophobe Beschichtung ist weniger aufwändig als eine hydrophobe Nanostrukturierung.

Die distale Seite ist die Seite, die zuerst in die Öffnung eingebracht wird. Die distale stirnseitige Fläche ist daher die Fläche, die zuerst mit dem Knochenmaterial in Kontakt kommt. Ist auch dieser Bereich hydrophob, kann sich Knochengewebe auch dort nicht ansiedeln. Damit wird die Hürde für ein Zuwachsen weiter erhöht, da Knochengewebe nicht einmal in die Nähe des Kanals gelangt. So kann eine möglichst dauerhafte Durchlässigkeit verbessert sichergestellt werden.

In einer Ausführungsform ist der Körper zweiteilig aufgebaut und umfasst einen Außenkörper und einen Innenkörper, der auch als Inlay bezeichnet werden kann und als Beschichtung des Außenkörpers dient. Der Außenkörper umgibt das Inlay und stellt die Außenseite mit dem Verankerungselement zur Verfügung. Das Inlay bildet den Kanal aus. Außenkörper und Inlay sind miteinander bevorzugt fest verbunden, und zwar insbesondere kraft-, form- und/oder stoffschlüssig. Bevorzugt ist das Inlay in den Außenkörper eingepresst und/oder mit diesem verklebt oder verschweißt.

Die Beschichtung ist mit anderen Worten durch Anordnen des Inlays im Außenkörper hergestellt. Ein Inlay kann beispielsweise einen kreisringförmigen Querschnitt aufweisen, wobei der Außendurchmesser des Inlays insbesondere zumindest ungefähr mit dem Innendurchmesser des im Außenkörper befindlichen Hohlraums entspricht. Das Inlay weist insbesondere zumindest ungefähr dieselbe Länge auf wie der Körper. Das Inlay umfasst insbesondere hydrophoben Kunststoff oder ist daraus hergestellt.

Bei einer Kombination einer hydrophoben Oberfläche oder mehrerer hydrophober Oberflächen mit einer geringen Querschnittsfläche kann besonders gut sichergestellt werden, dass ein Anwachsen dauerhaft verhindert wird.

In einer Ausgestaltung ist das Verankerungselement umlaufend angeordnet. Umlaufend bedeutet, dass ein Verankerungselement in Bezug auf die Längsachse über einen Winkel von 360° verläuft. Beispielsweise kann im Fall eines Körpers mit einem kreisringförmigen Querschnitt eine ebenfalls kreisringförmige, umlaufende Wulst an der Außenseite als Verankerungselement dienen. Das Verankerungselement kann rotationssymmetrisch ausgebildet sein.

In einer Ausgestaltung sind mehrere Verankerungselemente insbesondere gleichmäßig verteilt über die Außenseite angeordnet. Auch auf diese Weise kann ein Herausziehen oder Herausschieben gleichmäßig in axialer Richtung effektiv verhindert werden.

In einer Ausgestaltung sind mehrere separate, insbesondere umlaufende Verankerungselemente an unterschiedlichen Längenpositionen des Körpers axial voneinander beabstandet angeordnet.

Auf diese Weise wird das Gelenkimplantat an unterschiedlichen Positionen entlang der Längsachse sowie umlaufend in Bezug zur Längsachse gehalten. Damit wird ein Herausziehen oder Herausschieben besonders effektiv verhindert. Insbesondere sind die Verankerungselemente in gleichen Abständen angeordnet. Zwischen den Verankerungselementen wird die Außenseite typischerweise durch die Mantelfläche des Körpers ausgebildet.

Separate, voneinander beabstandete Verankerungselemente meint, dass die einzelnen Verankerungselemente räumlich voneinander getrennt sind. Zwischen den Verankerungselementen sind dann Lücken, in denen die Außenwand kein Verankerungselement aufweist. Ein umlaufendes Verankerungselement befindet sich somit typischerweise in einem definierten axialen Bereich. In einer benachbarten axialen Bereich befindet sich dann kein Verankerungselement. Dies gilt insbesondere für alle Winkelpositionen in Bezug zur Längsachse. Das Verankerungselement ist demnach nicht schraubenlinienförmig oder gewindeförmig ausgeführt.

Dies hat den Vorteil, dass zum Einbringen lediglich eine axiale Bewegung erfolgen muss. Ein kompliziertes Einschrauben ist nicht notwendig. Dies erleichtert das Setzen des Gelenkimplantats und verringert den technischen Aufwand der dafür benötigten Vorrichtung.

In einer Ausgestaltung ist das Verankerungselement als Widerhaken ausgestaltet. Der Widerhaken ist ein Haken, der rückwärtsgerichtet an dem Gelenkimplantat angebracht ist und dadurch verhindert, dass dieses herausgezogen oder herausgeschoben werden kann. Ein Herausziehen oder Herausschieben meint eine Bewegung des Gelenkimplantats entlang der Längsachse entgegen der Einbringrichtung. In dieser Ausgestaltung definiert das Verankerungselement die Einbringrichtung, entlang derer das Gelenkimplantat in eine Öffnung im Gelenkknochen eingebracht bzw. eingeschoben wird.

Beispielsweise hat das Verankerungselement eine schräge Außenfläche mit einem Winkel zwischen 90°, bevorzugt 110° und 180°, bevorzugt 160°, in Bezug zur der Oberfläche, die sich in Einbringrichtung hinter dem Verankerungselement befindet. Beim Einbringen kann das Verankerungselement einen Teil des die Öffnung ausbildenden Materials des Knochens verdrängen und/oder nach hinten gebogen werden (insbesondere im Falle eines Spreizhakens). Bei dem Versuch der Herausziehens oder Herausdrückens verkantet sich der äußere Rand des Verankerungselement im Material des Knochens und verhindert auf diese Weise die Bewegung. Das Verankerungselement kann beispielsweise in Form eines angewinkelten Stegs ausgebildet sein. Alternativ oder ergänzend kann das Verankerungselement als Spreizhaken ausgebildet sein.

Das Verankerungselement kann eine oder mehrere Schneidkanten umfassen, um Knochenmaterial zu schneiden. Auf diese Weise kann ein Einbringen erleichtert werden.

Mehrere in Einbringrichtung hintereinander angeordnete Verankerungselemente können als Rasthaken ausgebildet sein. Die Rasthaken können mit oder ohne einem Abstand zwischen einander angeordnet sein.

In einer Ausgestaltung befindet sich im Kanal mindestens ein beweglich angeordneter Scherkörper. Das Gelenkimplantat umfasst den Scherkörper. Ein Scherkörper ist ein Körper, der sich im Kanal bewegen kann, um dadurch beginnende Anhaftungen, beispielsweise von Proteinen und/oder Zellen, und/oder anwachsendes Gewebe abzuscheren und auf diese Weise weiter verbessert eine möglichst dauerhafte Durchlässigkeit des Kanals zu ermöglichen. Mit anderen Worten weist das Gelenkimplantat eine mechanisch wirkende Selbstreinigungsvorrichtung zur Reinigung des Kanals auf.

Ein Scherkörper kann beispielsweise die Form einer Kugel, eines Ovoids oder eines Zylinders wie z. B. eines Kreiszylinders aufweisen. Grundsätzlich sind auch unregelmäßig geformte Scherkörper möglich. Die Oberfläche des Scherkörpers ist insbesondere glatt und/oder hydrophob, um Anwachsungen am Scherkörper selbst zu verhindern. Runde Formen sind bevorzugt, um eine Drehung und damit eine verbesserte Selbstreinigung zu ermöglichen.

Insbesondere hat ein Scherkörper eine Dichte von wenigstens 4 g/cm³, bevorzugt wenigstens 6 g/cm³, besonders bevorzugt wenigstens 10 g/cm³ und in einer besonders bevorzugten Ausführungsform wenigstens 15 g/cm³ oder wenigstens 18 g/cm³. Je größer die Dichte, desto höher sind die erreichbaren Scherkräfte und damit die Wirksamkeit des Scherkörpers. Ein Scherkörper kann beispielsweise Tantal, eine Tantallegierung, Molybdän, Wolfram, Chrom-Vanadium-Stahl, Platin, eine Platinlegierung, Osmium, eine Osmiumlegierung, Gold, eine Goldlegierung, eine Eisenlegierung, eine Kobaltlegierung und/oder eine Nickellegierung umfassen oder daraus bestehen.

In einer Ausführungsform sind zwei Scherkörper vorhanden, die unabhängig voneinander beweglich sind. Durch die Reibung der beiden Scherkörper aneinander und insbesondere die Drehung der beiden Scherkörper können Ablagerungen verbessert gelöst werden.

Insbesondere ist ein Scherkörper axial beweglich, bevorzugt über wenigstens 50% und besonders bevorzugt wenigstens 75% der axialen Länge des Kanals. Auf diese Weise kann ein möglichst großer Bereich des Kanals gereinigt werden. Der Scherkörper kann sich bei Bewegung des Patienten, bei zyklischer Belastung und/oder durch die Wirkung der Schwerkraft im Kanal hin- und herbewegen und auf diese Weise Anlagerungen entfernen.

Es können zur Begrenzung der axialen Bewegung des Scherkörpers Halteelemente vorhanden sein, die insbesondere an der Innenseite der den Kanal ausbildenden Wandung angeordnet sind. Halteelemente können durch Verengungen, Vorsprünge oder sonstige Formelemente gebildet sein, die den Querschnitt lokal verringern und so eine Passage des Scherkörpers verhindern. Insbesondere sind im Bereich beider Enden des Kanals Halteelemente angeordnet. So wird sichergestellt, dass der oder die Scherkörper dauerhaft im Kanal verbleiben.

Der Scherkörper ist insbesondere so bemessen, dass zwischen dem Scherkörper und der den Kanal begrenzenden Innenwandung eine freie Querschnittsfläche von mindestens 0,2 mm² verbleibt, wie oben beschrieben. Beispielsweise kann im Falle eines Kanals mit kreisförmigem Querschnitt und eines Scherkörpers mit kreisförmigem Querschnitt der Durchmesser des Scherkörpers mindestens 20 µm kleiner sein als der Durchmesser des Kanals. Der Querschnitt sowie der Durchmesser werden hier bevorzugt in einer senkrecht zur Längsachse ausgerichteten Ebene gemessen.

In einer Ausführungsform ist der mindestens eine Scherkörper ferromagnetisch. Auf diese Weise kann von außen durch insbesondere periodisch wechselnde Magnetfelder eine Bewegung des Scherkörpers verursacht werden, infolge derer sich der mindestens eine Scherkörper bewegt und der Kanal gereinigt wird. Ein diesbezügliches Verfahren umfasst demnach das Ausüben von insbesondere periodisch wechselnden Magnetfeldern auf den oder die Scherkörper zwecks Bewegung des oder der Scherkörper im Kanal.

In einer Ausführungsform umfasst der mindestens eine Scherkörper ein Kernmaterial, welches insbesondere eine hohe Dichte aufweist, und eine das Kernmaterial insbesondere vollständig umgebende Beschichtung. Auf diese Weise kann ein korrosionsanfälliges, dichtes Kernmaterial verwendet werden und durch die Beschichtung ein dauerhafter Korrosionsschutz erzielt werden. Die Beschichtung kann ein inertes Metall, beispielsweise Platin, eine inerte Legierung oder einen Kunststoff umfassen oder daraus bestehen. Es ist auch möglich, einen oder mehrere Scherkörper durch insbesondere hochfrequente Magnetfelder durch Induktion von Wirbelströmen zu erhitzen und auf diese Weise Ablagerungen von z. B. Proteinen im Kanal zu lösen bzw. zu entfernen. Ein diesbezügliches Verfahren umfasst demnach das Aufbringen von insbesondere hochfrequenten Magnetfeldern auf den Körper und/oder den Scherkörper zwecks Erhitzen des Körpers bzw. des Scherkörpers.

In einer Ausführungsform beträgt ein Verhältnis des Durchmessers des Scherkörpers zur freien Bewegungslänge des Scherkörpers im Kanal mindestens 1:25, bevorzugt mindestens 1:20, besonders bevorzugt mindestens 1:15 und/oder höchstens 1:3, bevorzugt höchstens 1:5, besonders bevorzugt 1:8. Beispielsweise beträgt das Verhältnis 1:10. Im Falle eines Ovoids ist der größte Durchmesser gemeint. Auf diese Weise hat der mindestens eine Scherkörper genügend Spielraum für eine Bewegung im Kanal und damit durch eine Beschleunigung genügend kinetische Energie zum effektiven Abscheren von Verunreinigungen.

In einer Ausgestaltung ist ein freies Volumen des Kanals zumindest im Wesentlichen mit einem wasserlöslichen Füllstoff gefüllt. Ein wasserlöslicher Füllstoff dient dazu, dass beim Setzen oder Einbringen des Gelenkimplantats an seiner Zielposition im Knochen keine Knochensplitter oder Debris, die beispielsweise bei der Schaffung der Öffnung im Knochen etwa durch Mikrofrakturierung entstanden sind, in den Kanal gelangen. So wird ein Verstopfen des Kanals verhindert und eine Durchlässigkeit verbessert sichergestellt. Nach dem Setzen des Gelenkimplantats wird der Füllstoff durch die Wirkung von Körperflüssigkeit wie z. B. Blut herausgelöst und der Kanal somit geöffnet. Mit anderen Worten dient der Füllstoff als temporäres Verschlusselement.

Als Füllstoffe eignen sich grundsätzlich alle wasserlöslichen Stoffe, die keine ungewünschten biologischen Wirkungen haben. Bevorzugt enthält der Füllstoff einen oder mehrere Zuckeralkohole, ein oder mehrere Zuckeralkoholgemische, Gelatine und/oder Kollagen oder besteht daraus.

Das freie Volumen des Kanals kann auch dann mit dem Füllstoff gefüllt sein, wenn sich im Kanal zudem ein oder mehrere Scherkörper befinden.

Ein weiterer Aspekt der Erfindung ist eine Vorrichtung zum Setzen eines insbesondere erfindungsgemäßen Gelenkimplantats. Die Vorrichtung umfasst einen Griffbereich und einen Aufnahmebereich zur Aufnahme mindestens eines Gelenkimplantats. Der Aufnahmebereich umfasst einen Dorn zur Anordnung des Gelenkimplantats sowie eine Anlagefläche, an welcher das auf dem Dorn angeordnete Gelenkimplantat anliegen kann. Alle Merkmale, Vorteile und Ausgestaltungen des eingangs erwähnten Gelenkimplantats können auch für die Vorrichtung gelten und umgekehrt.

Ein erstes Ende des Dorns befindet sich im Bereich der Anlagefläche. Ein zweites Ende des Dorns ist ein freies Ende. Der Dorn ist insbesondere so ausgebildet, dass ein Gelenkimplantat über das freie Ende auf den Dorn geschoben werden kann, so dass das Gelenkimplantat an der Anlagefläche anliegt. Dies ist mit Anordnung des Gelenkimplantats gemeint und kann in Vorbereitung des Setzens des Gelenkimplantats erfolgen. Alternativ oder ergänzend kann die Vorrichtung bereits mit dem Gelenkimplantat bestückt sein. Die Vorrichtung kann dann am Griffbereich gegriffen werden. Die Vorrichtung wird relativ zum zu behandelnden Gelenkknochen so bewegt, dass der Dorn mitsamt dem Gelenkimplantat in die Öffnung bzw. Bohrung im Gelenkknochen eingebracht wird. Beim Einbringen kann eine ausgeübte vorwärts gerichtete Kraft über die Anlagefläche auf das Gelenkimplantat übertragen werden.

Der Dorn ist so ausgebildet, dass das Gelenkimplantat von dem Dorn über das freie Ende heruntergeschoben werden kann. Dies erfolgt nach dem Setzen des Gelenkimplantats. Die Vorrichtung mitsamt dem Dorn kann also wieder herausgezogen werden und das Gelenkimplantat verbleibt aufgrund des oder der Verankerungselement an seiner Position im Gelenkknochen.

Der Griffbereich dient insbesondere dem manuellen Greifen. Es ist jedoch nicht ausgeschlossen, dass der Griffbereich als Schnittstelle für ein maschinelles Greifen, etwa durch einen OP-Roboter, ausgebildet ist. Insbesondere befindet sich der Griffbereich an einem ersten Ende der Vorrichtung und der Aufnahmebereich befindet sich an einem zweiten Ende der Vorrichtung, das dem ersten Ende abgewandt ist.

Setzen meint das Einbringen des Gelenkimplantats, insbesondere in ein Gelenk, beispielsweise ein menschliches Gelenk wie z. B. ein Kniegelenk, Hüftgelenk oder Sprunggelenk. Das Gelenkimplantat wird in einen Gelenkknochen eingesetzt, insbesondere in eine Öffnung in einer subchondralen Knochenplatte.

Der Dorn führt das Gelenkimplantat und kann deshalb als Führungsdorn bezeichnet werden. Der Dorn hat insbesondere eine Querschnittsform, die der Querschnittsform des Kanals entspricht. Der Dorn hat, ausgehend von der Anlagefläche, eine Länge, die zumindest teilweise in den Kanal des Gelenkimplantats hineinreicht oder der Länge des Kanals entspricht.

In einer Ausführungsform ist die Länge des Dorns größer als die Länge des Kanals. Beim Setzen des Gelenkimplantats wird dann das freie Ende des Dorns zuerst ein Stück weit in die Öffnung im Knochen geschoben. Insbesondere ist das freie Ende des Dorns abgerundet und/oder weist im Vergleich zum sonstigen Durchmesser des Dorns einen verringerten Durchmesser auf. Auf diese Weise wird ein Einführen des Dorns in die Öffnung und/oder ein Aufstecken des Gelenkimplantats auf den Dorn erleichtert. Um Verletzungen zu verhindern, ist das freie Ende insbesondere nicht spitz ausgeführt.

Die Anlagefläche kann zum direkten oder indirekten Anliegen des Gelenkimplantats eingerichtet sein. Mit anderen Worten kann das zu setzende Gelenkimplantat direkt an der Anlagefläche anliegen, diese also direkt kontaktieren, oder indirekt daran anliegen. Im Falle der indirekten Anliegens liegen eine oder mehrere Teile zwischen der Anlagefläche und dem Gelenkimplantat, beispielsweise eine oder mehrere weitere bewegliche oder unbewegliche Komponenten der Vorrichtung und/oder eine oder mehrere weitere Gelenkimplantate.

Es ist vorteilhaft, wenn sich die Anlagefläche über im Wesentlichen die gesamte stirnseitige Fläche des Gelenkimplantats erstreckt. So werden Spannungen minimiert. Die Anlagefläche kann jedoch grundsätzlich auch als Anlagekante oder Anlagepunkt oder -punkte ausgebildet sein. Da auch in diesem Fall eine (wenn auch kleine) Fläche für die Anlage zur Verfügung steht, ist auch dies eine Anlagefläche im Sinne der Erfindung.

Der Griffbereich kann direkt an den Dorn anschließen. Alternativ kann zwischen Griffbereich und Dorn ein separater Bereich angeordnet sein, der beispielsweise zylinderförmig ausgebildet ist.

In einer Ausführungsform ist der Griffbereich länglich ausgebildet und geradlinig mit dem Dorn verbunden. Mit anderen Worten haben der Griffbereich und der Dorn eine gemeinsame Längsachse.

In einer alternativen Ausführungsform ist der insbesondere länglich ausgebildete Griffbereich winklig mit dem Dorn verbunden. Mit anderen Worten schneiden sich die Längsachse des Griffbereichs und des Dorns in einem Winkel. Der Winkel kann beispielsweise wenigstens 5°, bevorzugt wenigstens 10° oder 15° und/oder höchstens 45°, bevorzugt höchstens 35° oder 30° betragen. Je nach Operationstechnik kann die geradlinige oder winklige Ausgestaltung einen erleichterten Zugang zur Öffnung im Gelenkknochen ermöglichen.

In einer Ausgestaltung weist die Vorrichtung ein Haltemittel zum Halten des auf dem Dorn angeordneten Gelenkimplantats auf. Dies ermöglicht, dass das Gelenkimplantat während der Verwendung der Vorrichtung nicht unplanmäßig vom Dorn fällt. Auf diese Weise wird das Setzen des Implantats deutlich vereinfacht. Das Haltemittel ist insbesondere so ausgestaltet, dass das Gelenkimplantat nach dem Setzen im Knochen auf einfache Weise oder automatisch freigegeben wird, so dass der Dorn entfernt werden kann und das Gelenkimplantat im Knochen verbleibt.

In einer Ausgestaltung ist das Haltemittel durch eine zumindest bereichsweise Erweiterung des Dorns ausgestaltet. In einer Ausgestaltung ist das Haltemittel durch eine im Bereich des Dorns nach außen gerichtete Federkraft realisiert.

Der Dorn kann einen Außendurchmesser haben, der zumindest bereichsweise geringfügig größer ist als der Innendurchmesser des Kanals. Auf diese Weise wird das Gelenkimplantat verklemmt und auf diese Weise gehalten. Beispielsweise kann der Dorn in einem Bereich, der beispielsweise an die Anlagefläche angrenzt, leicht konisch geformt sein.

Alternativ oder ergänzend kann der Dorn zur Ausübung einer nach außen gerichteten Federkraft eingerichtet sein. Beispielsweise kann der Dorn in axialer Richtung geschlitzt sein und die einzelnen, zwischen den Schlitzen befindlichen Segmente sind aus einem federnden Material hergestellt und nach außen vorgespannt. Beim Aufstecken des Gelenkimplantats auf den Dorn können die Segmente entgegen der Federkraft nach radial innen bewegt werden.

Ein weiterer Aspekt der Erfindung ist eine Vorrichtung zum Setzen eines insbesondere erfindungsgemäßen Gelenkimplantats. Die Vorrichtung umfasst einen proximal angeordneten Haltebereich mit einem Griffteil und einem Betätigungselement. Die Vorrichtung umfasst einen sich entlang einer Längsachse in distale Richtung erstreckenden Hohlkörper zur Aufnahme zumindest eines Gelenkimplantats. Die Vorrichtung ist so eingerichtet, dass bei Betätigung des Betätigungselements zumindest ein im Hohlkörper befindliches Gelenkimplantat in distale Richtung bewegt wird. Alle Merkmale, Vorteile und Ausgestaltungen des eingangs erwähnten Gelenkimplantats sowohl der oben beschriebenen Vorrichtung können auch für diese Vorrichtung gelten und umgekehrt.

Der Haltebereich ist proximal angeordnet. Er ist so angeordnet, dass er von einer Ärztin oder einem Arzt gegriffen werden kann, um die Vorrichtung zu halten. Ein vom Haltebereich abgewandter, distaler Bereich der Vorrichtung dient zum Einführen des Gelenkimplantats in die Öffnung im Gelenkknochen.

Das Griffteil dient insbesondere dem manuellen Greifen. Es ist jedoch nicht ausgeschlossen, dass das Griffteil als Schnittstelle für ein maschinelles Greifen, etwa durch einen OP-Roboter, ausgebildet ist. Das Griffteil kann hohl sein.

Der Hohlkörper ist insbesondere im Inneren der Vorrichtung angeordnet. Insbesondere entspricht die Längsachse des Hohlkörpers der Längsachse des im Hohlkörper befindlichen Gelenkimplantats. Der Hohlkörper verläuft typischerweise bis zu einem distalen Ende der Vorrichtung. Der Hohlkörper kann sich bis in den Haltebereich erstrecken. Dies ist jedoch nicht notwendig. Der Hohlkörper umfasst typischerweise eine Wandung, die einen Hohlraum umgibt bzw. definiert. Der Hohlraum hat typischerweise einen Querschnitt, der dem äußeren Querschnitt des Gelenkimplantats entspricht. Der Hohlraum kann kreiszylinderförmig sein.

Die Längsachse des Hohlkörpers kann gerade oder zumindest bereichsweise gebogen sein. Die Vorrichtung kann steif oder entlang der Längsachse biegsam sein. Im Falle einer gebogenen oder biegsamen Längsachse gilt für einen Winkel der zwei gegenüber einander winklig angeordneten Teile insbesondere das oben für die Längsachse des Griffbereichs und die Längsachse des Dorns Gesagte analog.

Das Gelenkimplantat kann im Hohlkörper in distaler Richtung bewegt werden. Das Gelenkimplantat kann auf das distale Ende der Vorrichtung zu und/oder über das distale Ende der Vorrichtung hinaus bewegt werden. Das distale Ende des Hohlkörpers ist offen, damit Gelenkimplantate dort aus dem Hohlkörper herausbewegt werden können.

Das Betätigungselement löst eine Bewegung des Gelenkimplantats aus, wobei dies insbesondere eine Bewegung relativ zum übrigen Teil der Vorrichtung, beispielsweise zum Haltebereich ist. Insbesondere ist das Betätigungselement gegenüber dem Griffteil beweglich. So ist eine einfache Betätigung möglich. Das Betätigungselement ist insbesondere zur manuellen Betätigung eingerichtet, beispielsweise mit einem oder mehreren Fingern, allerdings ist jedoch auch eine maschinelle Betätigung nicht ausgeschlossen, etwa durch einen OP-Roboter. Das Betätigungselement kann proximal über das Griffteil hinausragen.

In einer Ausgestaltung ist distaler Bereich des Hohlkörpers als Halteeinrichtung zum Halten des Gelenkimplantats in einer Einbringposition eingerichtet.

Die Halteeinrichtung dient dem Halten eines Gelenkimplantats zum Einführen des Gelenkimplantats in die Öffnung des Gelenkknochens. Das Gelenkimplantat kann z. B. während einer Relativbewegung der Vorrichtung und während des Setzens des Gelenkimplantats von der Halteeinrichtung gehalten werden. Auf diese Weise wird verhindert, dass das Gelenkimplantat unplanmäßig herabfällt, bevor es seine Zielposition erreicht hat.

Das Halten kann während der Bewegung des Gelenkimplantats erfolgen. Die Bewegung des Gelenkimplantats kann das Setzen des Gelenkimplantats unterstützen. Das Halten muss kein im Bezug zur Vorrichtung unbewegliches Halten sein. Halten meint lediglich ein zumindest zeitweiliges Fixieren, um ein Herabfallen zu verhindern.

Die Halteeinrichtung kann dazu eingerichtet sein, das teilweise oder vollständig im Hohlkörper befindliche Gelenkimplantat zu halten. Es ist jedoch bevorzugt, dass sich das Gelenkimplantat während des Setzens zumindest teilweise, bevorzugt weitgehend, außerhalb des Hohlkörpers befindet. Dies erleichtert ein Setzen des Gelenkimplantats, da der Hohlkörper nicht in die Öffnung im Gelenkknochen eingebracht werden muss.

Insbesondere kann das Gelenkimplantat in distaler Richtung zumindest teilweise, insbesondere weitgehend und in einer Ausführungsform vollständig aus dem Hohlkörper heraus bewegt werden.

In einer Ausführungsform umfasst die Halteeinrichtung eine bereichsweise Erweiterung der den Hohlkörper definierenden Wandung nach innen, beispielsweise in Form einer oder mehrerer nach innen ragender Nasen oder eines umlaufenden, ringförmigen Stegs. Auf diese Weise kann eine Presspassung erreicht werden, mit der das Gelenkimplantat gehalten werden kann.

In einer Ausführungsform umfasst die Halteeinrichtung eine Einrichtung zur Ausübung einer insbesondere nach innen gerichteten Federkraft. Der Hohlkörper kann beispielsweise in axialer Richtung geschlitzt sein und die einzelnen, zwischen den Schlitzen befindlichen Segmente können aus einem federnden Material hergestellt und/oder nach innen vorgespannt sein. Es können beispielsweise zwei oder mehr Schlitze vorliegen. Schlitze verlaufen insbesondere ausgehend vom distalen Ende des Hohlkörpers. Es kann auch eine Kombination aus Schlitzen und Erweiterungen vorliegen. So kann ein besonders sicheres form- und/oder kraftschlüssiges Halten erreicht werden. Beim Ausüben einer Kraft durch das Betätigungselement kann die Haltekraft überwunden werden, um das Gelenkimplantat in distaler Richtung auszugeben.

In einer Ausgestaltung ist der Hohlkörper zur Aufnahme mehrerer axial hintereinander befindlicher Gelenkimplantate eingerichtet, welche bei Betätigung des Betätigungselements in distaler Richtung bewegt werden.

In diesem Fall ist die Vorrichtung dazu eingerichtet, ein Gelenkimplantat nach dem anderen in distaler Richtung zu bewegen und auf diese Weise aus dem distalen Ende des Hohlkörpers auszugeben. Die Vorrichtung dient damit als Dispenser für Gelenkimplantate.

Wird das am distalen Ende des Hohlkörpers befindliche Gelenkimplantat gehalten, werden damit auch automatisch die anderen Gelenkimplantate im Inneren des Hohlkörpers gehalten.

In einer Ausgestaltung weist die Vorrichtung ein mit dem Betätigungselement in Wirkverbindung stehendes Schubelement auf, um das Gelenkimplantat in distale Richtung zu bewegen.

Das Schubelement kann z. B. eine Schubstange sein. Das Schubelement kann massiv oder hohl sein. Insbesondere ist das Schubelement mechanisch mit dem Betätigungselement verbunden. Durch das Schubelement kann eine Bewegung des Betätigungselements auf das im Hohlkörper befindliche Gelenkimplantat übertragen werden. Die Bewegung kann hierbei manuell gezielt gesteuert werden. Das Schubelement ist insbesondere im Hohlkörper angeordnet und kontaktiert ein Gelenkimplantat, um dieses distal zu verschieben. Das Schubelement kann auf proximaler Seite beweglich im Hohlkörper angeordnet sein, um das Gelenkimplantat in distaler Richtung zu bewegen. Das Schubelement kann fest mit dem Betätigungselement verbunden oder verbindbar und/oder beweglich zum Betätigungselement angeordnet sein.

In einer Ausführungsform weist das Schubelement an seinem distalen Ende einen Dorn auf, der in dem Gelenkimplantat angeordnet werden kann. So wird die Position des Gelenkimplantats optimal gehalten und ein Verkanten oder Verdrehen weitestgehend ausgeschlossen.

In einer Ausgestaltung umfasst die Vorrichtung ein Federelement, mit dem das Betätigungselement direkt oder indirekt mit einer proximal gerichteten Federkraft beaufschlagbar ist.

Auf diese Weise kann das Betätigungselement nach erfolgter Betätigung wieder in seine Ausgangsposition zurückbewegt werden. Das Federelement kann dabei auch indirekt, also über ein oder mehrere andere Teile wie beispielsweise ein unten beschriebenes Schubteil, auf das Betätigungselement wirken.

Das Federelement kann als Spiralfeder ausgebildet und um das Schubteil herum angeordnet sein. Das Federelement kann sich auf Seiten des Schubteils an einem Abstützelement des Betätigungselements oder des Schubteils abstützen. Als Abstützelement kann beispielsweise eine Rückseite eines unten beschriebenen Anschlagelements, das die axiale Bewegung des Betätigungselements bzw. des Schubteils begrenzt, dienen. Das Abstützelement kann als mindestens teilweise umlaufender Steg ausgebildet sein. Das Federelement kann sich auf Seiten der Vorrichtung an einem Abstützelement im Inneren der Vorrichtung abstützen. Hier kann beispielsweise ein Absatz als Abstützelement dienen.

Das Betätigungselement oder das Schubteil kann ein Anschlagelement aufweisen, beispielsweise in Form eines insbesondere umlaufend, radial nach außen gerichteten Vorsprungs. Das Anschlagelement kann mit einem Teil der Vorrichtung zusammenwirken, um eine axiale Bewegung des Betätigungselements bzw. des Schubteils insbesondere in proximaler Richtung zu begrenzen. Das Anschlagelement kann als mindestens teilweise umlaufender Steg ausgebildet sein.

In einer Ausgestaltung weist das Betätigungselement ein Schubteil zum Bewegen des Schubelements auf. Insbesondere bewegt sich das Schubelement bei einer distal gerichteten Bewegung des Schubteils gemeinsam mit dem Schubteil. Insbesondere bewegt sich das Schubelement bei einer proximal gerichteten Bewegung des Schubteils nicht mit dem Schubteil.

Bei der proximal gerichteten Bewegung verbleibt das Schubelement insbesondere ortsfest in Bezug zum Griffteil und/oder zum Hohlkörper. Mit anderen Worten ist das Schubteil selektiv mit dem Schubelement koppelbar.

Dies ermöglicht bei mehrmaliger Betätigung des Betätigungselement jeweils ein Weiterbewegen des Schubelements und damit des oder der im Hohlkörper angeordneten Gelenkimplantate. So kann auf einfache Weise die wiederholte Ausgabe mehrerer Gelenkimplantate gewährleistet werden.

Schubteil und Schubelement können koaxial angeordnet sein. Das Schubteil kann hülsenförmig sein und das Schubelement kann im Inneren des Schubteils angeordnet sein. Auf diese Weise kann eine geradlinige und damit leichtgängige Kraftausübung gewährleistet werden.

Insbesondere umfasst das Schubteil und/oder das Schubelement Rastelemente wie beispielsweise keilförmige Nocken zum Herstellen einer zeitweisen kraft- und/oder formschlüssigen Kopplung.

Das Schubelement kann ein oder mehrere Rastelemente aufweisen, die eine Bewegung in distaler Richtung ermöglichen, aber eine Bewegung in proximaler Richtung verhindern. Die Rastelemente sind insbesondere auf einer distalen Seite spitzwinklig und weisen auf einer proximalen Seite eine Sperrfläche auf. Die Rastelemente können analog zu den Verankerungselementen des Gelenkimplantats ausgestaltet sein, weshalb auf die obige Beschreibung verwiesen wird, die hier analog gilt. Die axialen Abstände zwischen den Rastelementen können der axialen Länge des Gelenkimplantats entsprechen, so dass bei einer Betätigung genau ein Gelenkimplantat herausgedrückt wird.

Es kann am oder beim Schubteil ein erstes Gegenelement vorhanden sein, das die Rastelemente des Schubelements in distaler Richtung durchlässt und eine Passage in proximaler Richtung verhindert. Das Gegenelement kann das das Schubelement teilweise oder vollständig umgeben. Das Gegenelement kann beispielsweise in axialer Richtung geschlitzt sein und die einzelnen, zwischen den Schlitzen befindlichen Segmente können aus einem federnden Material hergestellt und/oder nach innen vorgespannt sein, um die Rastelemente in distaler Richtung durchzulassen.

Die Vorrichtung kann ein zweites Gegenelement aufweisen. Das zweite Gegenelement dient dazu, eine Bewegung des Schubelements in proximale Richtung zu blockieren, während das Schubteil mitsamt dem ersten Gegenelement in proximale Richtung bewegt wird. Das zweite Gegenelement ist insbesondere fest mit einem Gehäuse und/oder dem Griffteil der Vorrichtung verbunden. Die Verbindung kann direkt oder indirekt sein. Für das zweite Gegenelement gilt das oben für das erste Gegenelement Gesagte analog.

Die axiale Erstreckung des Hohlkörpers kann ein ganzzahliges Vielfaches der axialen Länge des Gelenkimplantats aufweisen. So kann eine bestimmte Anzahl der Gelenkimplantate in dem Hohlkörper angeordnet werden. In einer Ausführungsform können wenigstens 3, insbesondere wenigstens 4, bevorzugt wenigstens 5 und/oder höchstens 10, bevorzugt höchstens 8, insbesondere höchstens 6 oder 7 Gelenkimplantate im Hohlkörper aufgenommen werden.

In einer Ausführungsform ist ein distaler Bereich des Vorrichtung, insbesondere mit einer axialen Länge, die der axialen Länge wenigstens eines und bevorzugt zweier oder dreier Gelenkimplantate entspricht, aus einem transparenten oder transluzenten Material hergestellt. Dies betrifft insbesondere den Hohlkörper und ggf. auch darum herum angeordnete Teile z. B. eines Gehäuses der Vorrichtung. Auf diese Weise kann man optisch erkennen, ob noch eines oder mehrere Gelenkimplantate vorhanden sind.

Ein weiterer Aspekt der Erfindung ist ein System, umfassend mindestens ein insbesondere erfindungsgemäßes Gelenkimplantat und eine insbesondere erfindungsgemäße Vorrichtung zum Setzen des Gelenkimplantats. Alle Merkmale, Vorteile und Ausgestaltungen des eingangs erwähnten Gelenkimplantats sowie der oben beschriebenen Vorrichtungen können auch für das System gelten und umgekehrt.

Ein weiterer Aspekt der Erfindung ist ein Verfahren zum Setzen eines Gelenkimplantats mit einem durchgehenden Kanal, insbesondere eines erfindungsgemäßen Gelenkimplantats. Das Verfahren umfasst Einbringen des Gelenkimplantats in eine Öffnung, die eine subchondrale Knochenplatte durchdringt. Alle Merkmale, Vorteile und Ausgestaltungen des eingangs erwähnten Gelenkimplantats, der oben beschriebenen Vorrichtungen und des Systems können auch für das Verfahren gelten und umgekehrt.

Insbesondere umfasst der Verfahren ferner das Herstellen der Öffnung, beispielsweise durch Mikrofrakturierung, Perforieren und/oder Bohren. Insbesondere wird das Gelenkimplantat derart in die Öffnung eingebracht, dass der Kanal eine Seite der subchondralen Knochenplatte mit der anderen Seite der subchondralen Knochenplatte miteinander verbindet. Insbesondere dient das Verfahren zur Behandlung eines Knorpeldefekts. Insbesondere wird zum Einbringen eine Vorrichtung gemäß einem Aspekt der Erfindung verwendet. Insbesondere umfasst das Verfahren das Aufsetzen des Hohlkörpers der Vorrichtung und/oder des von der Vorrichtung gehaltenen Implantats auf den Implantationsort. Insbesondere umfasst das Verfahren das Bewegen des Gelenkimplantats in distaler Richtung und/oder das Herausziehen der Vorrichtung. Das Verfahren kann das mindestens teilweise Überdecken des Knorpeldefekts mit einer Membran, z. B. einer insbesondere zweischichtigen Kollagenmembran, umfassen.

Nachfolgend werden Aspekte und Ausführungsbeispiele der Erfindung auch anhand von Figuren näher erläutert.

Es zeigen:
- Figur 1:: eine Schnittzeichnung durch ein Gelenkimplantat;
- Figur 2:: eine perspektivische Schnittzeichnung eines Gelenkimplantats;
- Figur 3:: einen ersten Schritt eines Verfahrens;
- Figuren 4 bis 8:: weitere Schritte des Verfahrens;
- Figur 9:: ein Gelenkimplantat in seiner Bestimmungsposition;
- Figur 10:: ein erstes Ausführungsbeispiel einer Vorrichtung zum Setzen eines Gelenkimplantats;
- Figur 11:: ein zweites Ausführungsbeispiel einer Vorrichtung zum Setzen eines Gelenkimplantats;
- Figur 12:: ein drittes Ausführungsbeispiel einer Vorrichtung zum Setzen eines Gelenkimplantats;
- Figuren 13 bis 15:: Schritte bei der Verwendung einer Vorrichtung;
- Figur 16:: vergrößerten Details einer Vorrichtung.

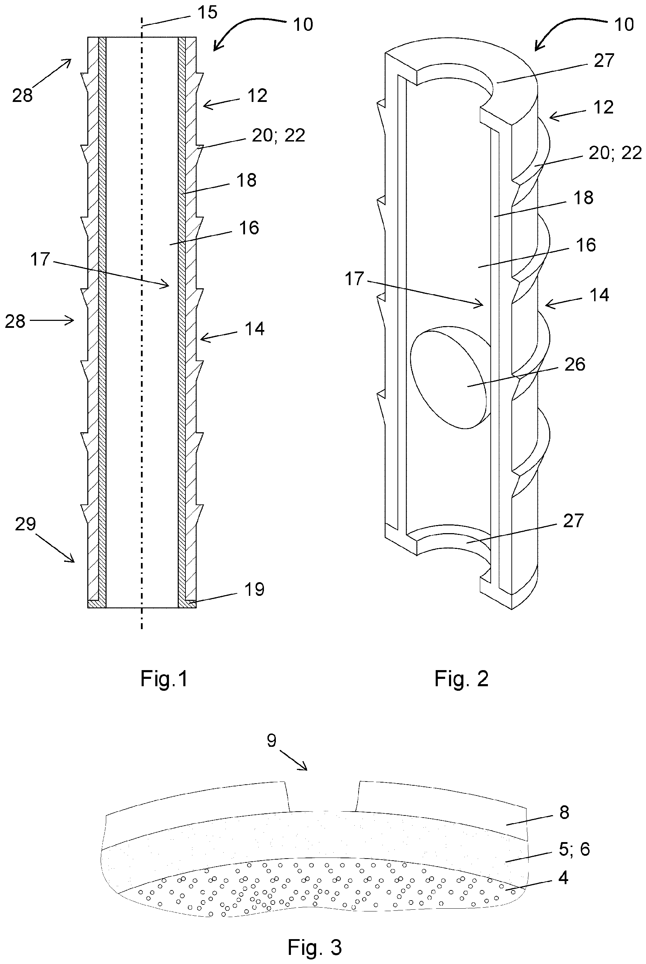

Die Figuren 1 und 2 zeigen Ausgestaltungen eines erfindungsgemäßen Gelenkimplantats 10. das Gelenkimplantat 10 ist als länglicher, rohrartiger Körper 12 ausgeformt, der sich um eine Längsachse 15 erstreckt. Der Körper 12 umschließt einen Kanal 16. Der Kanal 16 verläuft durchgehend von einem proximalen Ende 28 bis zu einem distalen Ende 29 des Gelenkimplantats 10.

Im hier gezeigten Beispiel hat der Körper 12 eine kreiszylinderförmige Grundform, der Kanal 16 hat die Form eines Kreiszylinders und das Gelenkimplantat 10 ist drehsymmetrisch in Bezug zur Längsachse 15.

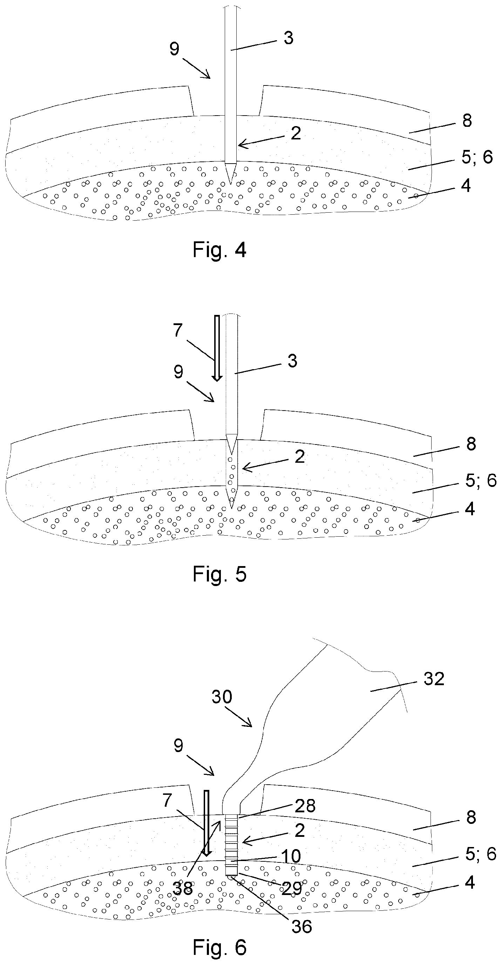

Das Gelenkimplantat 10 ist dafür vorgesehen, mit seinem distalen Ende 29 zuerst in eine Öffnung in einem Gelenkknochen eingeschoben zu werden (siehe Figur 6). Das Gelenkimplantat 10 umfasst mehrere Verankerungselemente 20, die hier beispielhaft als keilförmige Widerhaken 22 ausgeführt sind. Die Verankerungselemente 20 umfassen an ihrer distalen Seite jeweils eine schräge Außenfläche und sind an ihrer proximalen Seite als zumindest im Wesentlichen senkrecht zur Längsachse 15 ausgerichtete Stufen ausgeführt. Auf diese Weise wird beim Einschieben des Gelenkimplantats 10 das die Öffnung ausbildende Material des Knochens zeitweilig durch die schrägen Außenflächen verdrängt. Anschließend verhindern die stufenförmigen Strukturen ein Herausziehen des Gelenkimplantats 10 entgegen der Einbringrichtung. Auf diese Weise kann das Gelenkimplantat 10 mittels der Verankerungselemente 20 im Gelenkknochen verankert werden.

Im hier gezeigten Beispiel sind die Verankerungselemente 20 umlaufend sowie in regelmäßigen axialen Abständen angeordnet. Die Verankerungselemente 20 sind an unterschiedlichen Längenpositionen des Körpers 12 separat voneinander angeordnet.

Die Innenwand 17 und/oder eine distale stirnseitige Fläche 19 kann hydrophob ausgestaltet sein, um ein Einwachsen von Knochengewebe zu verhindern. Im hier gezeigten Ausführungsbeispiel sind eine den Kanal 16 definierende Innenwand 17 sowie die distale stirnseitige Fläche 19 mit einer hydrophoben Beschichtung 18 versehen. Die Beschichtung 18 kann beispielsweise als Inlay vorgesehen sein, sodass der Körper 12 aus zwei koaxialen Teilen zusammengesetzt ist. Alternativ kann die Beschichtung 18 beispielsweise durch ein Beschichtungsverfahren aufgetragen worden sein. Die Dicke der Beschichtung 18 in Bezug zum radial äußeren Bereich der Wandung kann beliebig gewählt sein.

Das Gelenkimplantat 10 aus Figur 2 ist dem Gelenkimplantat 10 der Figur 1 sehr ähnlich, sodass im Folgenden lediglich auf die Unterschiede eingegangen wird. Das Gelenkimplantat 10 umfasst einen Scherkörper 26 in Form einer Kugel, der frei im Kanal 16 beweglich ist. Bei Bewegung des Scherkörpers 26 im Kanal 16 können Anlagerungen an der Innenwand 17 so abgeschert werden.

An beiden axialen Enden des Gelenkimplantats 10 sind Halteelemente 27 in Form von hier beispielhaft umlaufenden Verengungen vorgesehen, die verhindern, dass der Scherkörper 26 den Kanal 16 verlässt. Die Verengungen sind in Form von Platten ausgestaltet und weisen jeweils ein insbesondere mittiges Durchgangsloch auf.

Figur 3 zeigt schematisch ein vergrößertes Detail eines beschädigten Gelenks. Dargestellt ist ein Gelenkknochen 5, nämlich eine subchondrale Platte 6. Darunter befindet sich das Knochenmark 4 im Bereich der Spongiosa, also eines durchbluteten Innenraums des Knochens. Oberhalb befindet sich ein Knorpel 8, der mittig eine Schädigung 9 aufweist. Beispielweise ist hier Knorpelgewebe örtlich zerstört oder fehlt.

Figur 4 zeigt, wie mit einem geeigneten Werkzeug 3 eine Öffnung 2 hergestellt wird, die durch die subchondrale Platte 6 hindurch verläuft. Beispielsweise wird hierzu das Werkzeug 3 entlang einer Einbringrichtung 7 durch die subchondrale Platte 6 hindurchgeschoben und/oder eine Öffnung gebohrt. Ein solches Vorgehen ist aus der Mikrofrakturierung bekannt und ermöglicht einen Transport von Blut und Knochenmark 4 durch die subchondrale Platte hindurch 6 in den Bereich der Schädigung 9 des Knorpels 8. in Figur 5 ist zu sehen, wie das Werkzeug 3 entgegen der Einbringrichtung 7 wieder herausgezogen wird und auf diese Weise die Öffnung 2 freigelegt wird. Knochenmark 4 beginnt, durch die Öffnung nach oben zu strömen.

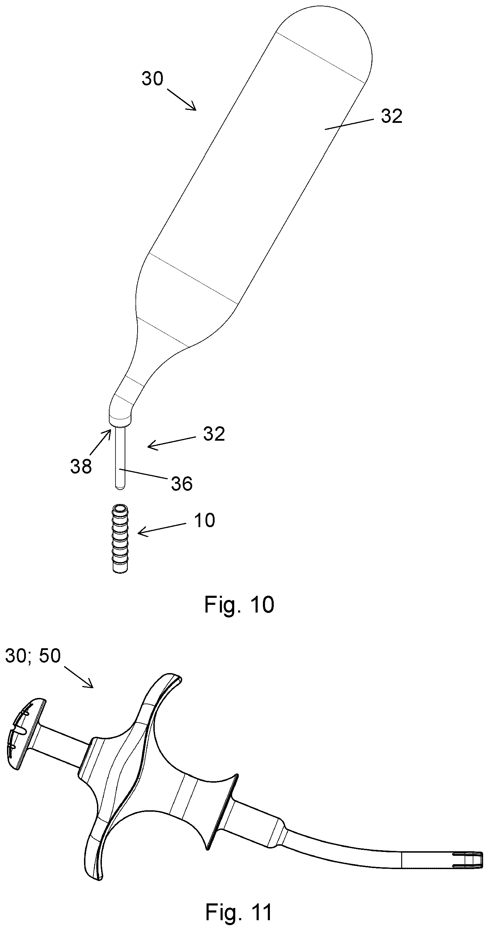

Figur 6 zeigt, wie anschließend - hier beispielhaft mit einer Vorrichtung 30 gemäß Figur 10 - ein Gelenkimplantat 10 in die Öffnung 2 eingebracht wird. Das Gelenkimplantat 10 wird entlang der Einbringrichtung 7, die in den gezeigten Darstellungen von oben nach unten verläuft, in die Öffnung 2 eingeschoben. Dabei wird das Gelenkimplantat 10 mit seinem distalen Ende 29 zuerst in die Öffnung 2 eingeschoben, und zwar insbesondere derart, dass das proximale Ende 28 etwa bündig mit der Oberkante der subchondralen Platte 6 abschließt. Das distale Ende 29 kann dann über die Unterkante der subchondralen Platte 6 hinaus ragen.

Die Vorrichtung 30, die in Figur 10 vollständig dargestellt ist, umfasst einen Griffbereich 32 und einen Aufnahmebereich 32 mit einem Dorn 36, auf den das Gelenkimplantat 10 während des Einbringens und insbesondere auch schon zuvor aufgeschoben ist. Es ist eine typischerweise senkrecht zum Dorn ausgerichtete, bevorzugt um den Dorn 36 herum verlaufende Anlagefläche 38 vorhanden, an der die proximale Stirnseite des Gelenkimplantats 10 während des Einbringens anliegen kann. Bevorzugt befinden sich am Dorn 36 Haltemittel dem Halten des Gelenkimplantats 10, wie beispielsweise eine Erweiterung und/oder eine Einrichtung zur Ausübung einer nach außen gerichteten Federkraft.

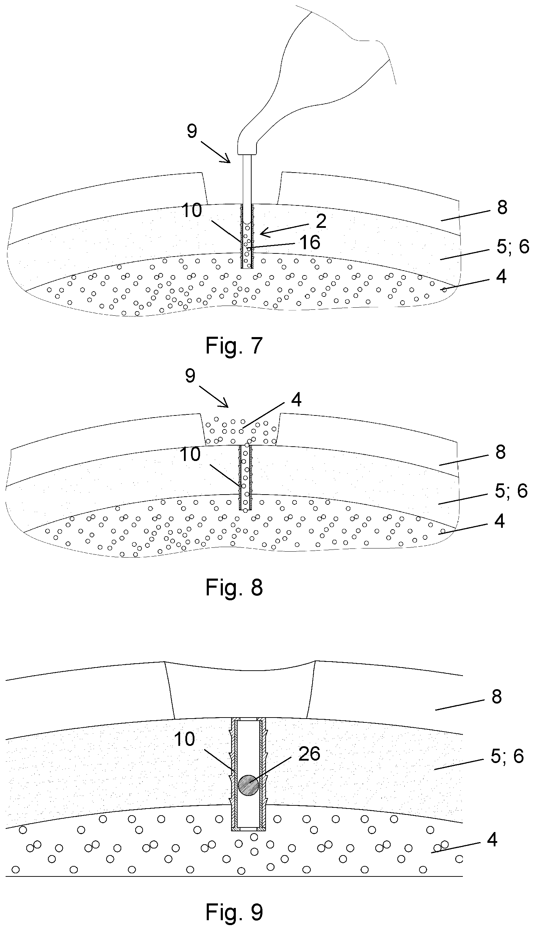

Die Figuren 7 und 8 zeigen in Schnittdarstellungen, dass die Vorrichtung 30 entgegen der Einbringrichtung herausgezogen wird und dass Knochenmark 4 von unten durch den Kanal 16 hindurch in den Bereich der Schädigung 9 strömt.

Figur 9 zeigt vergrößert eine alternative Ausgestaltung, beispielsweise die Ausgestaltung aus Figur 2, eines Gelenkimplantats 10 in seiner Zielposition in der Öffnung 2. Hierbei ist im Inneren des Gelenkimplantats 10 ein kugelförmiger Scherkörper 26 angeordnet.

Figur 11 zeigt eine Vorrichtung 30 oder 50 mit einem links dargestellten Bereich zum manuellen Greifen und einem rechts dargestellten distalen Bereich zum Halten und/oder Bewegen eines Gelenkimplantats zwecks Setzens des Gelenkimplantats. Es ist sichtbar, dass ein länglich ausgebildeter Bereich, der sich zwischen einem axialen Mittelpunkt und dem rechts dargestellten distalen Ende der Vorrichtung 30 oder 50 beispielsweise mittig befindet, gebogen sein kann. Bei allen anderen dargestellten Vorrichtungen 30 ist dieser Bereich üblicherweise gradlinig ausgebildet. Die hier gezeigte, gebogene Ausführungsform kann mit allen anderen Ausführungsformen von Vorrichtungen 30 oder 50 kombiniert werden.

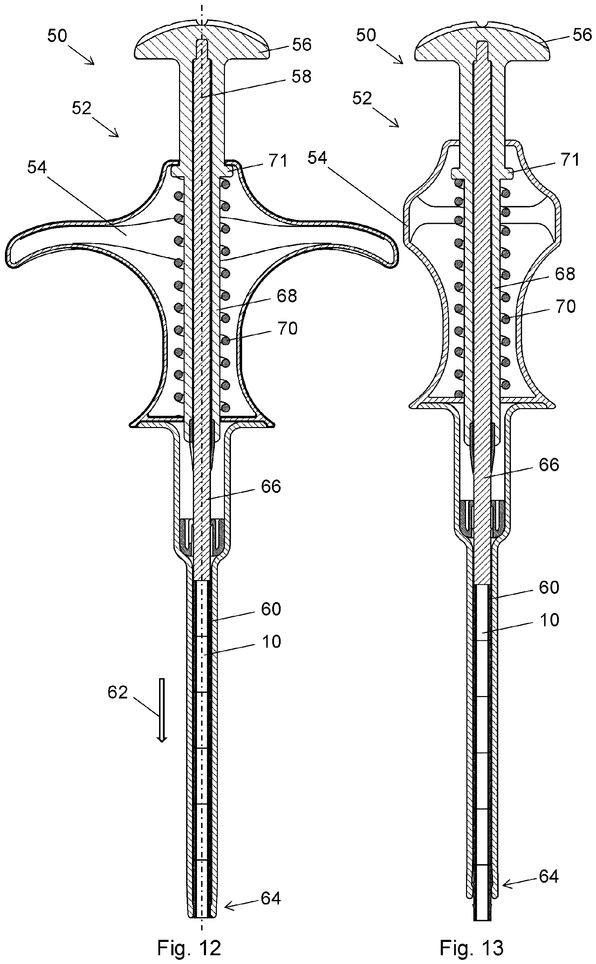

Figur 12 zeigt eine Vorrichtung 50 zum Setzen von Gelenkimplantaten 10. Die Vorrichtung umfasst einen proximal befindlichen Haltebereich 52, der von einem Benutzer manuell gegriffen werden kann. Dazu umfasst der Haltebereich 52 ein Griffteile 54. Ferner umfasst der Halterbereich ein Betätigungselement 56, das hier als mechanisch wirkender Druckknopf ausgebildet ist, der in axialer Richtung in Bezug zur Längsachse 58 der Vorrichtung 50 gedrückt werden kann. die Vorrichtung 50 umfasst ferner einen Hohlkörper 60, der sich durch zumindest einen Teil der Vorrichtung hindurch axial in distale Richtung 62 bis zum distalen Ende der Vorrichtung 50 erstreckt. Der Hohlkörper 60 ist hier zentral angeordnet und hat einen kreisförmigen Querschnitt. In dem Hohlkörper befindet sich mindestens ein Gelenkimplantat 10, hier beispielhaft mehrere hintereinander angeordnete Gelenkimplantate 10. Die Vorrichtung umfasst ferner ein Schubelement 66, mit dem das oder die Gelenkimplantate 10 in distale Richtung 62 bewegt werden können. Das Schubelement 66 steht in Wirkverbindung mit dem Betätigungselement 56, so dass die Bewegung des Schubelements 66 durch Betätigung des Betätigungselements 56 ausgelöst werden kann. In einem einfachen Fall können das Betätigungselement 56 und das Schubelement 66 fest miteinander verbunden oder einstückig ausgebildet sein.

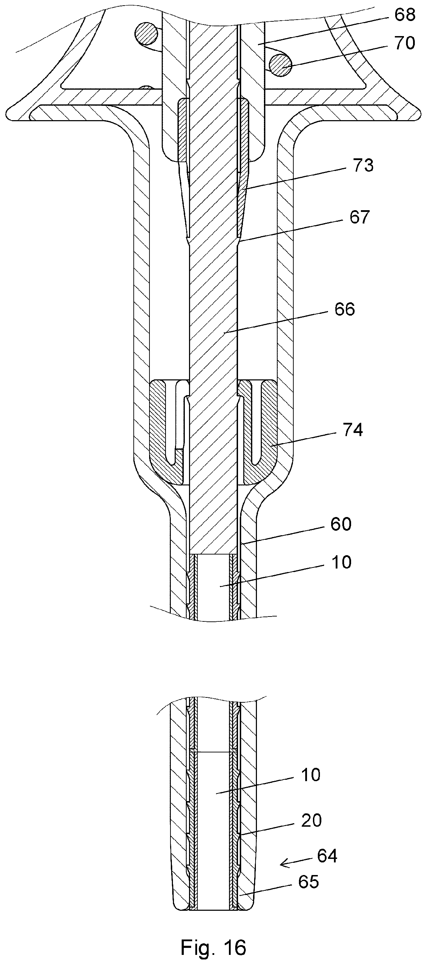

Der distale Bereich bzw. das distale Ende des Hohlkörpers ist als Halteeinrichtung 64 ausgestaltet, die dazu dient, das dort befindliche Gelenkimplantat 10 gegen ein unplanmäßiges Herausfallen zu sichern. Die Halteeinrichtung ist in Figur 16 vergrößert dargestellt. Die den Hohlkörper 60 definierende Wandung weist eine nach innen ragende Erweiterung 65 auf, die eine Presspassung mit der Außenwandung des Gelenkimplantats 10 ermöglicht. Insbesondere ist eine axiale Länge der Erweiterung 65 höchstens so lang wie der axiale Abstand zwischen zwei benachbarten Verankerungselementen 20 des Gelenkimplantats. Bevorzugt entspricht die Länge der Erweiterung 65 in etwa der Länge des Zwischenraums zwischen zwei Verankerungselementen 20, so dass das Gelenkimplantat 10 in einer definierten Position gehalten wird.

Figur 12 zeigt weiterhin, dass die Vorrichtung 50 ein Schubteil 68 aufweist, welches hier beispielhaft fest mit dem Betätigungselement 56 verbunden oder einstückig mit diesem ausgeführt ist. Das Schubteil 68 dient dazu, die Bewegung in distale Richtung des Betätigungselement 56 auf das Schubelement 66 zu übertragen. Das Schubteil 68 ist hier rohrförmig ausgestaltet und umgibt das Schubelement 66.

Die Vorrichtung 50 weist ferner ein Federelement 70 auf, um das Betätigungselement 56 in proximale Richtung zu drücken. Das Federelement 70 ist zwischen einem umlaufenden Anschlagelement 71 am Schubteil 68 und einem distalen Abschnitt des Gehäuses der Vorrichtung 50 eingespannt. Die obere Seite des Anschlagelements 71 begrenzt die Bewegung des Schubteils 68 bzw. des Betätigungselements 56 in proximaler Richtung.

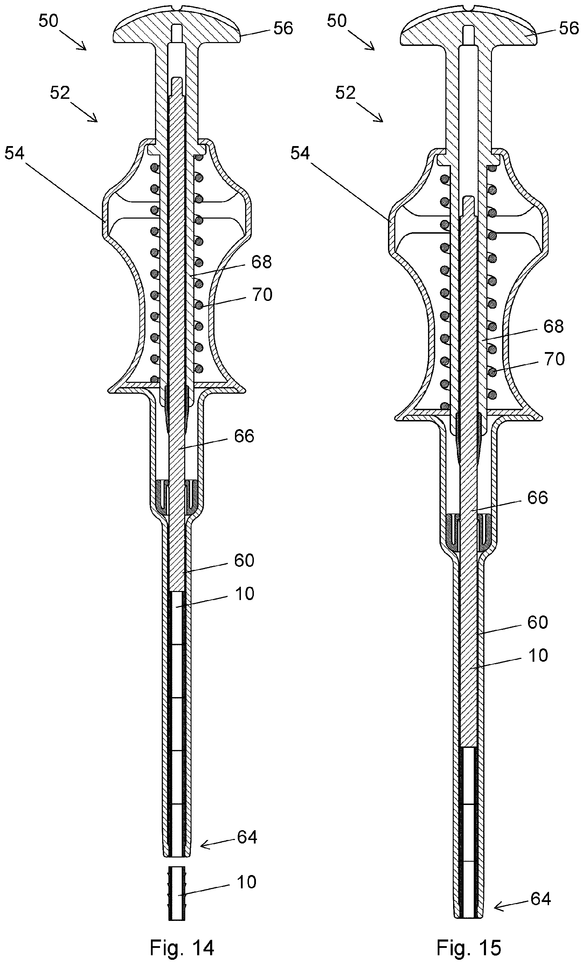

Figur 13 zeigt die Vorrichtung 50 in einer um 90° gedrehten Ausrichtung und in einer Lage, in der das Betätigungselement 56 gegen die Federkraft betätigt wird. Das Federelement 70 ist komprimiert und das Anschlagelement 71 ist in distaler Richtung von dem proximalen Gehäuseteil beabstandet. Das Schubelement 66 wird gemeinsam mit dem Betätigungselement 56 in distale Richtung gedrückt und bewegt das unterste Gelenkimplantat 10 distal aus der Halteeinrichtung 64 heraus. Die vollständig herausgedrückte Position des Gelenkimplantats 10 ist in Figur 14 dargestellt. Aufgrund der Federkraft ist das Betätigungselement 56 wieder in die Ausgangsposition zurück bewegt worden. Dabei ist allerdings das Schubelement 66 in der um die Länge eines Gelenkimplantats 10 weiter distal befindlichen Position geblieben. Mit anderen Worten ist die Vorrichtung 50 so eingerichtet, dass eine Betätigung des Betätigungselement 56 eine Bewegung des Schubteils 68 und infolgedessen auch des Schubelements 66 in distale Richtung verursacht. Eine entgegengesetzte Bewegung des Betätigungselements 56 und/oder des Schubteils 68 führt hingegen nicht zu einer Bewegung des Schubelements 66. Eine beispielhafte Umsetzung dieser Funktionalität ist in Figur 16 dargestellt, die vergrößerte Details der Vorrichtung 50 zeigt.

Das Schubelement 66 umfasst Rastelemente 67, die als umlaufende und/oder geneigte Nocken ausgebildet sein können. Die Vorrichtung 50 umfasst ein erstes Gegenelement 73, das am Schubteil 68 befestigt ist, zumindest teilweise um das Schubelement 66 herum angeordnet ist und zumindest bereichsweise in radiale Richtung elastisch beweglich ist. Das erste Gegenelement 73 ermöglicht eine Bewegung des Schubelements 66 in distaler Richtung relativ zum Schubteil 68 und verhindert eine Bewegung des Schubelements 66 in proximaler Richtung relativ zum Schubteil 68.

Beispielsweise ist das erste Gegenelement 73 von unten in axialer Richtung geschlitzt, so dass die einzelnen Segmente sich elastisch nach außen bewegen können, um bei Bewegung des Schubelements 66 in distaler Richtung 62 die Rastelemente 67 passieren zu lassen. Eine Bewegung in proximaler Richtung wird durch die oberen Flächen der Rastelemente 67, die insbesondere in radialer Richtung ausgerichtet sind, verhindert, wie es in Figur 16 gezeigt ist. Zu diesem Zweck weist das erste Gegenelement 73 eine typischerweise ebenfalls radial ausgerichtete Unterseite auf.

Die Vorrichtung 50 umfasst ein zweites Gegenelement 74, das zumindest teilweise um das Schubelement 66 herum angeordnet ist und zumindest bereichsweise in radiale Richtung elastisch beweglich ist. Das zweite Gegenelement 74 kann ebenfalls in axialer Richtung geschlitzt sein. Das zweite Gegenelement 74 ist an einem Gehäuse der Vorrichtung 50 befestigt und ermöglicht eine Bewegung des Schubelements 66 in distaler Richtung relativ zum Gehäuse bzw. zum Hohlkörper 60 und verhindert eine Bewegung des Schubelements 66 in proximaler Richtung relativ zum Gehäuse bzw. zum Hohlkörper 60. Das zweite Gegenelement 74 weist eine Sperrfläche auf, um die radial ausgerichtete Oberseite der Rastelemente 67 gegen eine Bewegung in proximale Richtung zu sperren, wie dies in Figur 16 gezeigt ist.

Jedes der Gegenelemente 73, 74 kann im proximalen Bereich einen größeren Innendurchmesser bzw. einen Abstand zum Schubelement 66 aufweisen, um ein Einführen der keilförmigen Rastelemente 67 zu erleichtern.

Beim Betätigen des Betätigungselements 56 bewegt sich so das Schubelement 66 gemeinsam mit dem Schubteil 68 und beim Loslassen des Betätigungselements 56 wird nur das Betätigungselement 56 mit dem Schubteil 68 zurückbewegt, während das Schubelement 66 in der distalen Position verbleibt. Die axialen Abstände zwischen den Rastelementen 67 am Schubelement entsprechen insbesondere der axialen Länge des Gelenkimplantats, so dass das Schubelement 66 immer genau um ein Implantat-Länge vorgeschoben wird, um ein Gelenkimplantat aus dem Hohlkörper 60 herauszubewegen.

Figur 15 zeigt eine Situation, in der bereits mehrere Gelenkimplantate 10 in distaler Richtung aus dem Hohlkörper 60 herausbewegt worden sind. Das Schubelement 66 ist entsprechend in distaler Richtung verschoben. Das Betätigungselement 56 ist hingegen in seiner Ausgangsposition, so dass ein weiteres Gelenkimplantat herausbewegt werden kann.

In jeder der hier gezeigten Ausführungsformen der Vorrichtung 50, insbesondere in Fällen, in denen nur ein Gelenkimplantat im Hohlkörper 60 ist, kann im Hohlkörper 60 ein zentraler, axial ausgerichteter Stab oder Dorn vorhanden sein, der in dem Kanal des im Hohlkörper 60 befindlichen Gelenkimplantats 10 angeordnet ist. Ein solcher Stab oder Dorn reicht insbesondere bis zum distalen Ende des Hohlkörpers oder überragt dieses in distaler Richtung.

**Bezugszeichenliste**

| | |
|---|---|
| Öffnung | 2 |
| Werkzeug | 3 |
| Knochenmark | 4 |
| Gelenkknochen | 5 |
| Subchondrale Platte | 6 |
| Einbringrichtung | 7 |
| Knorpel | 8 |
| Schädigung | 9 |
| Gelenkimplantat | 10 |
| Körper | 12 |
| Außenseite | 14 |
| Längsachse | 15 |
| Kanal | 16 |
| Innenwand | 17 |
| Beschichtung | 18 |
| Stirnseitige Fläche | 19 |
| Verankerungselement | 20 |
| Widerhaken | 22 |
| Scherkörper | 26 |
| Halteelement | 27 |
| Proximales Ende | 28 |
| Distales Ende | 29 |
| Vorrichtung | 30 |
| Griffbereich | 32 |
| Aufnahmebereich | 34 |
| Dorn | 36 |
| Anlagefläche | 38 |
| Vorrichtung | 50 |
| Haltebereich | 52 |
| Griffteil | 54 |
| Betätigungselement | 56 |
| Längsachse (d Vorrichtung) | 58 |
| Hohlkörper | 60 |
| distale Richtung | 62 |
| Halteeinrichtung | 64 |
| Erweiterung | 65 |
| Schubelement | 66 |
| Rastelemente | 67 |
| Schubteil | 68 |
| Federelement | 70 |
| Anschlagelement | 71 |
| Erstes Gegenelement | 73 |
| Zweites Gegenelement | 74 |

## Patentansprüche

1. Gelenkimplantat (10) zum Freihalten einer Öffnung (2) in einem Gelenkknochen (5), umfassend einen länglichen Körper (12), der sich entlang einer Längsachse (15) erstreckt, wobei der Körper (12) entlang der Längsachse (15) einen durchgehenden Kanal (16) definiert, wobei an einer Außenseite (14) des Körpers (12) mindestens ein Verankerungselement (20) zum Verankern des Gelenkimplantats (10) in dem Gelenkknochen (5) angeordnet ist.

2. Gelenkimplantat (10) nach dem vorhergehenden Anspruch, **dadurch gekennzeichnet, dass** der Kanal (16) an einer engsten Stelle eine freie Querschnittsfläche von kleiner oder gleich 0,2 mm² aufweist.

3. Gelenkimplantat (10) nach einem der vorhergehenden Ansprüche, **dadurch gekennzeichnet, dass** eine den Kanal (16) definierende Innenwand (17) des Körpers (12) und/oder eine distale stirnseitige Fläche (19) des Körpers (12) mit einer hydrophoben Beschichtung versehen ist.

4. Gelenkimplantat (10) nach einem der vorhergehenden Ansprüche, **dadurch gekennzeichnet, dass** das Verankerungselement (20) umlaufend angeordnet ist und/oder dass mehrere Verankerungselemente (20) gleichmäßig verteilt über die Außenseite angeordnet sind.

5. Gelenkimplantat (10) nach einem der vorhergehenden Ansprüche, **dadurch gekennzeichnet, dass** mehrere separate, umlaufende Verankerungselemente (20) an unterschiedlichen Längenpositionen des Körpers (12) axial voneinander beabstandet angeordnet sind.

6. Gelenkimplantat (10) nach einem der vorhergehenden Ansprüche, **dadurch gekennzeichnet, dass** das Verankerungselement (20) als Widerhaken (22) ausgestaltet ist.

7. Gelenkimplantat (10) nach einem der vorhergehenden Ansprüche, **dadurch gekennzeichnet, dass** sich im Kanal (16) mindestens ein beweglich angeordneter Scherkörper (26) befindet.

8. Gelenkimplantat (10) nach einem der vorhergehenden Ansprüche, **dadurch gekennzeichnet, dass** ein freies Volumen des Kanals (16) zumindest im Wesentlichen mit einem wasserlöslichen Füllstoff gefüllt ist.

9. Vorrichtung (30) zum Setzen eines Gelenkimplantats (10) gemäß einem der Ansprüche 1 bis 8, umfassend einen Griffbereich (32) und einen Aufnahmebereich (34) zur Aufnahme mindestens eines Gelenkimplantats (10), wobei der Aufnahmebereich (34) einen Dorn (36) zur Anordnung des Gelenkimplantats (10) sowie eine Anlagefläche (38) umfasst, an welcher das auf dem Dorn (36) angeordnete Gelenkimplantat (10) anliegen kann.

10. Vorrichtung (30) nach dem vorhergehenden Anspruch, **dadurch gekennzeichnet, dass** die Vorrichtung (30) ein Haltemittel zum Halten des auf dem Dorn (36) angeordneten Gelenkimplantats (10) aufweist.

11. Vorrichtung (30) nach dem vorhergehenden Anspruch, **dadurch gekennzeichnet, dass** das Haltemittel durch eine zumindest bereichsweise Erweiterung des Dorns (36) und/oder eine im Bereich des Dorns (36) nach außen gerichtete Federkraft ausgestaltet ist.

12. Vorrichtung (50) zum Setzen eines Gelenkimplantats (10) gemäß einem der Ansprüche 1 bis 8, umfassend einen proximal angeordneten Haltebereich (52) mit einem Griffteil (54) und einem Betätigungselement (56), einen sich entlang einer Längsachse (58) in distale Richtung (62) erstreckenden Hohlkörper (60) zur Aufnahme zumindest eines Gelenkimplantats (10), wobei die Vorrichtung (50) so eingerichtet ist, dass bei Betätigung des Betätigungselements (56) zumindest ein im Hohlkörper (60) befindliches Gelenkimplantat (10) in distale Richtung (62) bewegt wird.

13. Vorrichtung (50) nach dem vorhergehenden Anspruch, **dadurch gekennzeichnet, dass** ein distaler Bereich des Hohlkörpers (60) als Halteeinrichtung (64) zum Halten des Gelenkimplantats (10) in einer Einbringposition eingerichtet ist.

14. Vorrichtung (50) nach einem der zwei vorhergehenden Ansprüche, **dadurch gekennzeichnet, dass** der Hohlkörper (60) zur Aufnahme mehrerer axial hintereinander befindlicher Gelenkimplantate (10) eingerichtet ist, welche bei Betätigung des Betätigungselements (56) in distaler Richtung (62) bewegt werden.

15. Vorrichtung (50) nach einem der drei vorhergehenden Ansprüche, **dadurch gekennzeichnet, dass** die Vorrichtung (50) ein mit dem Betätigungselement (56) in Wirkverbindung stehendes Schubelement (66) aufweist, um das Gelenkimplantat (10) in distale Richtung (62) zu bewegen.

16. Vorrichtung (50) nach einem der vier vorhergehenden Ansprüche, **dadurch gekennzeichnet, dass** die Vorrichtung (50) ein Federelement (70) umfasst, mit dem das Betätigungselement (56) mit einer proximal gerichteten Federkraft beaufschlagbar ist.

17. Vorrichtung (50) nach einem der zwei vorhergehenden Ansprüche, **dadurch gekennzeichnet, dass** das Betätigungselement (56) ein Schubteil (68) zum Bewegen des Schubelements (66) aufweist, wobei sich das Schubelement (66) bei einer distal gerichteten Bewegung des Schubteils (68) gemeinsam mit dem Schubteil (68) bewegt, wohingegen sich das Schubelement (66) bei einer proximal gerichteten Bewegung des Schubteils (68) nicht mit dem Schubteil (68) bewegt.

18. System, umfassend mindestens ein Gelenkimplantat (10) gemäß einem der Ansprüche 1 bis 8 und eine Vorrichtung (30) zum Setzen des Gelenkimplantats (10) gemäß einem der Ansprüche 9 bis 11 oder eine Vorrichtung (50) zum Setzen des Gelenkimplantats (10) gemäß einem der Ansprüche 12 bis 16.

19. Verfahren zum Setzen eines Gelenkimplantats (10) mit einem durchgehenden Kanal (16), insbesondere gemäß einem der Ansprüche 1 bis 8, umfassend:
- Einbringen des Gelenkimplantats (10) in eine Öffnung (2), die eine subchondrale Knochenplatte durchdringt.
